# EUROPEAN PATENT SPECIFICATION

(11) **EP 2 620 453 B1**
(45) Date of publication and mention of the grant of the patent: **15.03.2017**
(21) Application number: 11826306.0
(22) Date of filing: 09.09.2011
(51) Int. Cl.: C07K 19/00, A61P 31/04, C12N 15/62, A61K 48/00

(54) **FUSION PROTEIN SAMB, CODING GENE AND APPLICATION THEREOF**
FUSIONSPROTEIN SAMB, FÜR DIESES KODIERENDES GEN UND ANWENDUNG DAVON
PROTÉINE DE FUSION SAMB, SON GÈNE CODANT ET SON APPLICATION

(30) Priority: 25.09.2010 CN 201010290471
(43) Date of publication of application: 31.07.2013
(73) Proprietor: Institute of Microbiology and Epidemiology, Academy of Military Medical Sciences, PR China, Fengtai District Beijing 100071 (CN)
(72) Inventor: WANG, Hui, Beijing 100071 (CN); CAI, Kun, Beijing 100071 (CN); LI, Tao, Beijing 100071 (CN); HOU, Xiaojun, Beijing 100071 (CN); WANG, Qin, Beijing 100071 (CN); GAO, Xiang, Beijing 100071 (CN)
(74) Representative: De Vries & Metman
(86) International application number: PCT/CN2011/001542
(87) International publication number: WO 2012/037779

(56) References cited:
- EP-A1- 1 057 895
- CN-A- 1 631 438
- CN-A- 101 062 410
- CN-A- 101 240 019
- CN-A- 101 314 616
- CN-A- 101 629 158
- CN-A- 101 948 546
- DATABASE JPO Proteins [Online] 24 August 2012 (2012-08-24), "JP 2009527226-A/25: Shiga Toxoid Chimeric Proteins.", XP002720449, retrieved from EBI accession no. JPOP:DD923320 Database accession no. DD923320 -& JP 2009 527226 A 30 July 2009 (2009-07-30) -& WO 2007/098201 A2 (HENRY M JACKSON FOUNDATION [US]; SMITH MICHAEL J [US]; O'BRIEN ALISON) 30 August 2007 (2007-08-30)
- SMITH M J ET AL: "Development of a hybrid Shiga holotoxoid vaccine to elicit heterologous protection against Shiga toxins types 1 and 2", VACCINE, ELSEVIER LTD, GB, vol. 24, no. 19, 8 May 2006 (2006-05-08), pages 4122-4129, XP028010812, ISSN: 0264-410X, DOI: 10.1016/J.VACCINE.2006.02.035 [retrieved on 2006-05-08]
- GAO X ET AL: "Immunogenicity of a novel Stx2B-Stx1B fusion protein in a mice model of Enterohemorrhagic Escherichia coli O157:H7 infection", VACCINE, ELSEVIER LTD, GB, vol. 27, no. 14, 23 March 2009 (2009-03-23), pages 2070-2076, XP025969109, ISSN: 0264-410X, DOI: 10.1016/J.VACCINE.2009.01.115 [retrieved on 2009-02-02]
- BAO TUZHONG ET AL.: 'The recombinant expression and receptor-binding activity of the B subunit of Shiga-like toxin type II' CHINA BIOTECHNOLOGY vol. 28, no. 10, 2008, pages 23 - 27

### SEQUENCE LISTING

<110> Institute of microbiology and epidemiology, Academy of military medical sciences, PR China
<120> Fusion Protein SAmB, Coding Gene and Application Thereof
<150> CN201010290471.2
   <151> 2010-09-25
<160> 21
<170> BiSSAP 1.2
<210> 1
   <211> 891
   <212> DNA
   <213> Artificial Sequence
<220>
   <221> source
   <222> 1..891
   <223> /mol_type="unassigned DNA" /note="chemically synthesized" /organism="Artificial Sequence"
<400> 1
   cgggagttta cgatagactt ttcgacccaa caaagttatg tctcttcgtt aaatagtata      60
   cggacagaga tatcgacccc tcttgaacat atatctcagg ggaccacatc ggtgtctgtt     120
   attaaccaca ccccaccggg cagttatttt gctgtggata tacgagggct tgatgtctat     180
   caggcgcgtt ttgaccatct tcgtctgatt attgagcaaa ataatttata tgtggccggg     240
   ttcgttaata cggcaacaaa tactttctac cgtttttcag attttacaca tatatcagtg     300
   cccggtgtga caacggtttc catgacaacg gacagcagtt ataccactct gcaacgtgtc     360
   gcagcgctgg aacgttccgg aatgcaaatc agtcgtcact cactggtttc atcatatctg     420
   gcgttaatgg agttcagtgg taatacaatg accagagatg catccagagc agttctgcgt     480
   tttgtcactg tcacagcaga agccttacgt ttcaggcaga tacagagaga atttcgtcag     540
   gcactgtctg aaactgctcc tgtgtatacg atgacgccgg gagacgtgga cctcactctg     600
   aactgggggc gaatcagcaa tgtgcttccg gagtatcggg gagaggatgg tgtcagagtg     660
   gggagaatat cctttaataa tatatcagcg atactgggga ctgtggccgt tatactgaat     720
   tgccatcatc agggggcgcg ttctgttcgc gccgtgaatg aagagagtca accagaatgt     780
   cagataactg gcgacaggcc tgttataaaa ataaacaata cattatggga aagtaataca     840
   gctgcagcgt ttctgaacag aaagtcacag tttttatata caacgggtaa a              891
<210> 2
   <211> 207
   <212> DNA
   <213> Artificial Sequence
<220>
   <221> source
   <222> 1..207
   <223> /mol_type="unassigned DNA" /note="chemically synthesized" /organism="Artificial Sequence"
<400> 2
   acgcctgatt gtgtaactgg aaaggtggag tatacaaaat ataatgatga cgataccttt     60
   acagttaaag tgggtgataa agaattattt accaacagat ggaatcttca gtctcttctt    120
   ctcagtgcgc aaattacggg gatgactgta accattaaaa ctaatgcctg tcataatgga    180
   gggggattca gcgaagttat ttttcgt                                        207
<210> 3
   <211> 1113
   <212> DNA
   <213> Artificial Sequence
<220>
   <221> source
   <222> 1..1113
   <223> /mol_type="unassigned DNA" /note="chemically synthesized" /organism="Artificial Sequence"
<400> 3
   cgggagttta cgatagactt ttcgacccaa caaagttatg tctcttcgtt aaatagtata      60
   cggacagaga tatcgacccc tcttgaacat atatctcagg ggaccacatc ggtgtctgtt     120
   attaaccaca ccccaccggg cagttatttt gctgtggata tacgagggct tgatgtctat     180
   caggcgcgtt ttgaccatct tcgtctgatt attgagcaaa ataatttatc agtggccggg     240
   ttcgttaata cggcaacaaa tactttctac cgtttttcag attttacaca tatatcagtg     300
   cccggtgtga caacggtttc catgacaacg gacagcagtt ataccactct gcaacgtgtc     360
   gcagcgctgg aacgttccgg aatgcaaatc agtcgtcact cactggtttc atcatatctg     420
   gcgttaatgg agttcagtgg taatacaatg accagagatg catccagagc agttctgcgt     480
   tttgtcactg tcacagcaca agccttatta ttcaggcaga tacagagaga atttcgtcag     540
   gcactgtctg aaactgctcc tgtgtatacg atgacgccgg gagacgtgga cctcactctg     600
   aactgggggc gaatcagcaa tgtgcttccg gagtatcggg gagaggatgg tgtcagagtg     660
   gggagaatat cctttaataa tatatcagcg atactgggga ctgtggccgt tatactgaat     720
   tgccatcatc agggggcgcg ttctgttcgc gccgtgaatg aagagagtca accagaatgt     780
   cagataactg gcgacaggcc tgttataaaa ataaacaata cattatggga aagtaataca     840
   gctgcagcgt ttctgaacag aaagtcacag tttttatata caacgggtaa aggaggagga     900
   ggttctacgc ctgattgtgt aactggaaag gtggagtata caaaatataa tgatgacgat     960
   acctttacag ttaaagtggg tgataaagaa ttatttacca acagatggaa tcttcagtct    1020
   cttcttctca gtgcgcaaat tacggggatg actgtaacca ttaaaactaa tgcctgtcat    1080
   aatggagggg gattcagcga agttattttt cgt                                 1113
<210> 4
   <211> 371
   <212> PRT
   <213> Artificial Sequence
<220>
   <223> Chemically synthesized
<400> 4
   Arg Glu Phe Thr Ile Asp Phe Ser Thr Gln Gln Ser Tyr Val Ser Ser
   1               5                   10                  15
   Leu Asn Ser Ile Arg Thr Glu Ile Ser Thr Pro Leu Glu His Ile Ser
               20                  25                  30
   Gln Gly Thr Thr Ser Val Ser Val Ile Asn His Thr Pro Pro Gly Ser
           35                  40                  45
   Tyr Phe Ala Val Asp Ile Arg Gly Leu Asp Val Tyr Gln Ala Arg Phe
       50                  55                  60
   Asp His Leu Arg Leu Ile Ile Glu Gln Asn Asn Leu Ser Val Ala Gly
   65                  70                  75                  80
   Phe Val Asn Thr Ala Thr Asn Thr Phe Tyr Arg Phe Ser Asp Phe Thr
                   85                  90                  95
   His Ile Ser Val Pro Gly Val Thr Thr Val Ser Met Thr Thr Asp Ser
               100                 105                 110
   Ser Tyr Thr Thr Leu Gln Arg Val Ala Ala Leu Glu Arg Ser Gly Met
           115                 120                 125
   Gln Ile Ser Arg His Ser Leu Val Ser Ser Tyr Leu Ala Leu Met Glu
       130                 135                 140
   Phe Ser Gly Asn Thr Met Thr Arg Asp Ala Ser Arg Ala Val Leu Arg
   145                 150                 155                 160
   Phe Val Thr Val Thr Ala Gln Ala Leu Leu Phe Arg Gln Ile Gln Arg
                   165                 170                 175
   Glu Phe Arg Gln Ala Leu Ser Glu Thr Ala Pro Val Tyr Thr Met Thr
               180                 185                 190
   Pro Gly Asp Val Asp Leu Thr Leu Asn Trp Gly Arg Ile Ser Asn Val
           195                 200                 205
   Leu Pro Glu Tyr Arg Gly Glu Asp Gly Val Arg Val Gly Arg Ile Ser
       210                 215                 220
   Phe Asn Asn Ile Ser Ala Ile Leu Gly Thr Val Ala Val Ile Leu Asn
   225                 230                 235                 240
   Cys His His Gln Gly Ala Arg Ser Val Arg Ala Val Asn Glu Glu Ser
                   245                 250                 255
   Gln Pro Glu Cys Gln Ile Thr Gly Asp Arg Pro Val Ile Lys Ile Asn
               260                 265                 270
   Asn Thr Leu Trp Glu Ser Asn Thr Ala Ala Ala Phe Leu Asn Arg Lys
           275                 280                 285
   Ser Gln Phe Leu Tyr Thr Thr Gly Lys Gly Gly Gly Gly Ser Thr Pro
       290                 295                 300
   Asp Cys Val Thr Gly Lys Val Glu Tyr Thr Lys Tyr Asn Asp Asp Asp
   305                 310                 315                 320
   Thr Phe Thr Val Lys Val Gly Asp Lys Glu Leu Phe Thr Asn Arg Trp
                   325                 330                 335
   Asn Leu Gln Ser Leu Leu Leu Ser Ala Gln Ile Thr Gly Met Thr Val
               340                 345                 350
   Thr Ile Lys Thr Asn Ala Cys His Asn Gly Gly Gly Phe Ser Glu Val
           355                 360                 365
   Ile Phe Arg
       370
<210> 5
   <211> 297
   <212> PRT
   <213> Artificial Sequence
<220>
   <223> chemically synthesized
<400> 5
   Arg Glu Phe Thr Ile Asp Phe Ser Thr Gln Gln Ser Tyr Val Ser Ser
   1               5                   10                  15
   Leu Asn Ser Ile Arg Thr Glu Ile Ser Thr Pro Leu Glu His Ile Ser
               20                  25                  30
   Gln Gly Thr Thr Ser Val Ser Val Ile Asn His Thr Pro Pro Gly Ser
           35                  40                  45
   Tyr Phe Ala Val Asp Ile Arg Gly Leu Asp Val Tyr Gln Ala Arg Phe
       50                  55                  60
   Asp His Leu Arg Leu Ile Ile Glu Gln Asn Asn Leu Tyr Val Ala Gly
   65                  70                  75                  80
   Phe Val Asn Thr Ala Thr Asn Thr Phe Tyr Arg Phe Ser Asp Phe Thr
                   85                  90                  95
   His Ile Ser Val Pro Gly Val Thr Thr Val Ser Met Thr Thr Asp Ser
               100                 105                 110
   Ser Tyr Thr Thr Leu Gln Arg Val Ala Ala Leu Glu Arg Ser Gly Met
           115                 120                 125
   Gln Ile Ser Arg His Ser Leu Val Ser Ser Tyr Leu Ala Leu Met Glu
       130                 135                 140
   Phe Ser Gly Asn Thr Met Thr Arg Asp Ala Ser Arg Ala Val Leu Arg
   145                 150                 155                 160
   Phe Val Thr Val Thr Ala Glu Ala Leu Arg Phe Arg Gln Ile Gln Arg
                   165                 170                 175
   Glu Phe Arg Gln Ala Leu Ser Glu Thr Ala Pro Val Tyr Thr Met Thr
               180                 185                 190
   Pro Gly Asp Val Asp Leu Thr Leu Asn Trp Gly Arg Ile Ser Asn Val
           195                 200                 205
   Leu Pro Glu Tyr Arg Gly Glu Asp Gly Val Arg Val Gly Arg Ile Ser
       210                 215                 220
   Phe Asn Asn Ile Ser Ala Ile Leu Gly Thr Val Ala Val Ile Leu Asn
   225                 230                 235                 240
   Cys His His Gln Gly Ala Arg Ser Val Arg Ala Val Asn Glu Glu Ser
                   245                 250                 255
   Gln Pro Glu Cys Gln Ile Thr Gly Asp Arg Pro Val Ile Lys Ile Asn
               260                 265                 270
   Asn Thr Leu Trp Glu Ser Asn Thr Ala Ala Ala Phe Leu Asn Arg Lys
           275                 280                 285
   Ser Gln Phe Leu Tyr Thr Thr Gly Lys
       290                 295
<210> 6
   <211> 42
   <212> DNA
   <213> Artificial Sequence
<220>
   <221> source
   <222> 1..42
   <223> /mol_type="unassigned DNA" /note="chemically synthesized" /organism="Artificial Sequence"
<400> 6
   cgggagttta cgatagactt ttcgacccaa caaagttatg tc 42
<210> 7
   <211> 58
   <212> DNA
   <213> Artificial Sequence
<220>
   <221> source
   <222> 1..58
   <223> /mol_type="unassigned DNA" /note="Chemically synthesized" /organism="Artificial Sequence"
<400> 7
   acaatcaggc gtagaacctc ctcctccttt acccgttgta tataaaaact gtgacttt 58
<210> 8
   <211> 45
   <212> DNA
   <213> Artificial Sequence
<220>
   <221> source
   <222> 1..45
   <223> /mol_type="unassigned DNA" /note="Chemically synthesized" /organism="Artificial Sequence"
<400> 8
   taaaggagga ggaggttcta cgcctgattg tgtaactgga aaggt 45
<210> 9
   <211> 39
   <212> DNA
   <213> Artificial Sequence
<220>
   <221> source
   <222> 1..39
   <223> /mol_type="unassigned DNA" /note="Chemically synthesized" /organism="Artificial Sequence"
<400> 9
   acgaaaaata acttcgctga atccccctcc attatgaca 39
<210> 10
   <211> 36
   <212> DNA
   <213> Artificial Sequence
<220>
   <221> source
   <222> 1..36
   <223> /mol_type="unassigned DNA" /note="Chemically synthesized" /organism="Artificial Sequence"
<400> 10
   tattaacgaa cccggccact gataaattat tttgct 36
<210> 11
   <211> 36
   <212> DNA
   <213> Artificial Sequence
<220>
   <221> source
   <222> 1..36
   <223> /mol_type="unassigned DNA" /note="Chemically synthesized" /organism="Artificial Sequence"
<400> 11
   agcaaaataa tttatcagtg gccgggttcg ttaata 36
<210> 12
   <211> 33
   <212> DNA
   <213> Artificial Sequence
<220>
   <221> source
   <222> 1..33
   <223> /mol_type="unassigned DNA" /note="Chemically synthesized" /organism="Artificial Sequence"
<400> 12
   tatctgcctg aataataagg cttgtgctgt gac 33
<210> 13
   <211> 33
   <212> DNA
   <213> Artificial Sequence
<220>
   <221> source
   <222> 1..33
   <223> /mol_type="unassigned DNA" /note="Chemically synthesized" /organism="Artificial Sequence"
<400> 13
   gtcacagcac aagccttatt attcaggcag ata 33
<210> 14
   <211> 55
   <212> DNA
   <213> Artificial Sequence
<220>
   <221> source
   <222> 1..55
   <223> /mol_type="unassigned DNA" /note="Chemically synthezised" /organism="Artificial Sequence"
<400> 14
   aagtgcagtt taataatgac ggaggaggag gttctcctga ttgtgtaact ggaaa 55
<210> 15
   <211> 30
   <212> DNA
   <213> Artificial Sequence
<220>
   <221> source
   <222> 1..30
   <223> /mol_type="unassigned DNA" /note="Chemically synthesized" /organism="Artificial Sequence"
<400> 15
   gcggccgcac gaaaaataac ttcgctgaat 30
<210> 16
   <211> 40
   <212> DNA
   <213> Artificial Sequence
<220>
   <221> source
   <222> 1..40
   <223> /mol_type="unassigned DNA" /note="Chemically synthesized" /organism="Artificial Sequence"
<400> 16
   ccccccccat atggctaaag gtaaaattga gttttccaag 40
<210> 17
   <211> 42
   <212> DNA
   <213> Artificial Sequence
<220>
   <221> source
   <222> 1..42
   <223> /mol_type="unassigned DNA" /note="Chemically synthesized" /organism="Artificial Sequence"
<400> 17
   gtcattatta aactgcactt cagcaaatcc ggagcctgat tc 42
<210> 18
   <211> 36
   <212> DNA
   <213> Artificial Sequence
<220>
   <221> source
   <222> 1..36
   <223> /mol_type="unassigned DNA" /note="Chemically synthesized" /organism="Artificial Sequence"
<400> 18
   ggggggaatt catgaagtgt atattattta aatggg 36
<210> 19
   <211> 56
   <212> DNA
   <213> Artificial Sequence
<220>
   <221> source
   <222> 1..56
   <223> /mol_type="unassigned DNA" /note="Chemically synthesized" /organism="Artificial Sequence"
<400> 19
   ttttttgtcg acttagtggt ggtggtggtg gtggtcatta ttaaactgca cttcag 56
<210> 20
   <211> 49
   <212> DNA
   <213> Artificial Sequence
<220>
   <221> source
   <222> 1..49
   <223> /mol_type="unassigned DNA" /note="Chemically synthesized" /organism="Artificial Sequence"
<400> 20
   ggggggggaa ttcatgaaaa taattatttt tagagtgcta acttttttc 49
<210> 21
   <211> 49
   <212> DNA
   <213> Artificial Sequence
<220>
   <221> source
   <222> 1..49
   <223> /mol_type="unassigned DNA" /note="Chemically synthesized" /organism="Artificial Sequence"
<400> 21
   gtcgactcag tggtggtggt ggtggtgacg aaaaataact tcgctgaat 49

## Description

### Field of the Invention

The present invention relates to a fusion protein SAmB which is associated with the prevention and/or treatment of infections caused by Shiga toxin-producing pathogenic bacteria, its coding gene and the use thereof.

### Technical background

The infection caused by enterohemorrhagic *Escherichia coli* (EHEC) O157: H7 is a newly developed foodborne infectious disease, which has become a global health problem. This infection mainly causes severe complications such as infantile diarrhea, hemorrhagic colitis (HC), hemolytic uremic syndrome (HUS) and thrombotic thrombocytopenic purpura (TTP). The infection may also lead to death. Since first explosively found in USA in 1982, the outbreaks of EHEC O157:H7 infection were found in Japan, USA, England, Canada, Australia etc., especially during May to August in 1996, in China, EHEC O157: H7 *E. Coli.* have been isolated from human, livestocks and other animals in Fujian, Gansu, Zhejiang, Anhui and other provinces since the first infection was reported in Xuzhou in 1986. In 2001, infectious diarrhea caused by EHEC O157: H7 broke out epidemically in Jiangsu and Anhui provinces, resulting in more than 20000 patients of which 177 died.

Attention has been made to EHEC O157: H7 infection by professionals and scientists all over the world because of its explosively epidemic trend, its severe pathogenicity and its fatality and the fact that if an antibiotic treatment is applied, it will make the condition even worse. Retrospective studies on EHEC O157: H7 infection have shown that the infection may stimulate immunity. This is reflected by the following facts, the occurrence of EHEC O157: H7-associated diarrhea becomes less frequent with age, and the ratio of symptomatic EHEC infections to asymptomatic infections decreased with age. Further, the relationship between the primary EHEC O157: H7 infection and subsequent infections with similar toxin and colonization factor (showing a protecting effect) also illustrates the effect of infection on immunity strengtherns the above facts. Up to now, there is no available EHEC O157: H7 vaccine for human yet but many vaccine candidates are being studied. For example, the whole molecule or the partial structure of some components such as the adhesion - associated factor, toxin and hemolysin have become the most important targets to be screened as protective antigens.

The toxin Stx family mainly includes type-I and type-II Stxs (Stx1 and Stx2), two members without immunological cross-reactions. An EHEC O157: H7 strain may express either one of Stx1 or Stx2 or both Stx1 and Stx2. Though Stx1 and Stx2 have different antigenicity, Stx1 and Stx2 have the same molecular structure and receptor(s) as well as similar toxicity mechanism. In particular, Stx1 and Stx2 are all composed of one A subunit and five B subunits. The A subunit has intracellular toxicity and binds to 28S rRNA to stop the protein synthesis, which is the underlying pathological mechanism of the clinical manifestation caused by EHEC O157: H7 infection. The B subunit has a cell-binding property and binds to cells having a specific receptor, the globotriaos-ylceramide (Gb3) or globotatrasylceramide (Gb4) so as to activate the enzymatic activity of the A subunit. Most EHEC O157: H7 strains contain at least Stx2 and Stx2 has a higher toxicity than Stx1. Clinical studies have revealed that Stx is closely related to severe infection complications such as HUS and TTP and is the key factor resulting in EHEC O157: H7 infection-caused renal injury or even death.

The B subunit of Stx is a protective antigen and has several effective epitopes. Boyd et al. used the purified Stx B subunit or synthesized fragment of B subunit to provide a protective effect to inoculated rabbits against the attack of Stx1 with a lethal dose. Marcato P et al. has suggested that Stx2B is not toxic to Vero cells and does not induce apoptosis of Burkitt's lymphoma cells. Further, the specific anti-Stx2B antibody generated in inoculated rabbits fights against the Stx2 with a lethal dose. The pre-study by present inventors also has revealed that the cloned and expressed Stx B subunit and its polypeptide fragments have no toxicity and provide good immune protection. Futher, the polyclonal antibodies for Stx1B and Stx2B could block the activity of the toxins but cannot provide protection against the strains producing both Stx1B and Stx2B (Bao, Shizhong et al. Bulletin of the Academy of Military Medical Sciences, 2008,32(6):537-540; Gao et al., Vaccine 2009,27:2070-2076). The previous studies of the inventors have also revealed that Stx2B-Stx1B fusion protein (simply referred to as 2S) constructed based on Stx1B and Stx2B could induce the mice to generate antibodies respectively against type-I and type-II Shiga toxins but the neutralization titers are type-dependent. For example, the neutralization titer was relatively low for Stx2, with the expression being that the protection ratio for the immunized animals decreased when the amount of bacteria (lysate) was higher than the lethal dose. Therefore, in order to improve
the immune protection level against dual-type toxins, this invention is aimed to select novel and more effective protective antigen components to build up a novel fusion protein which may be developed into a novel vaccine having better protective effects.

Some experiments implied that an inactivated antigen of the Stx2 A subunit is a good antigen and may induce antibodies with a high titer in vivo. A relevant study reported that the Stx2 A subunit was inactivated by site-directed mutagenesis at enzyme activity-associated Tyr77 (Ser77), Glul67 (Glnl67) and Argl70 (Lysl70) so as to construct a Stx2 mutant. Different from the natural toxin, the obtained recombinant toxin as such did not have cytotoxicity in Hela cells and could induce the mice to generation of immune antibodies or naturalization antibodies with a high titer in mice (O' Brien et al., Vaccine, 2006). However, as these recombinant toxins mimicked the structures of the natural ones (1A:5B) having one A subunit and five B subunits, it is hard to prepare them due to the structure assembly during the expression process. Such process cannot be performed in large scale and the obtained proteins are neither stable nor easy to store, which limits the application of the above-mentioned method. In addition, it has been reported that StxB was an effective biological adjuvant having an immune-facilitating effect and thus can be used as an intramolecular adjuvant to improve the immune efficacy of a new fusion protein so that a better protective effect could be obtained (Nacilla H et al., Int Immunol 2003; Nacilla H et al., J Immunol 2000). Up to now, a vaccine that is safer, more effective and has a wider protection scope is still needed against the infection of *Escherichia coli* 0157. WO2007/098201 discloses a chimeric Shiga toxoid containing an enzymatically-inactivated StxA2 subunit and the native StxB1 subunit. It aims at preparing a holotoxoid with the same structure as natural Stx, i.e with a AB5 composition. From the Western Blot assay it is clear that two subunits have been prepared rather than one fused protein. Smith M.J. et Al. Vaccine, Vol. 24 (19) 2006 discloses a chimeric Shiga toxoid for the development of a hybrid StxA2/StxB 1 holotoxoid vaccine to elicit heterologous protection against shiga toxins types 1 and 2. Also in this document no real fusion protein is obtained as is evidenced by the two bands that are present in the Western Blot assay.

### Summary of the Invention

The present invention aims to provide a fusion protein.

The fusion protein provided in the present invention is named SAmB and comprises at the N-terminal a Shiga toxin type-II A subunit mutant protein and at the C-terminal a Shiga toxin type-I B subunit, wherein the amino sequence is represented by SEQ ID NO.4 in the Sequence List.

The above-mentioned Shiga toxin type-II A subunit mutant protein is a protein obtained by mutating amino acid residues in the active site in the Shiga toxin type-II A subunit.

Furthermore, the above-mentioned amino acid residues in the active site are residue 77, 167 and 170 in the Shiga toxin type-II A subunit. The amino acid sequence of the Shiga toxin type-II A subunit is represented by SEQ ID NO. 5 in the Sequence List.

More specifically, the amino acid sequence of the Shiga toxin type-II A subunit mutant protein is represented by amino acids 1-297 of SEQ ID NO. 4 in the Sequence List. The amino acid sequence of the B subunit of type-I Shiga toxin is represented by animo acids 303-371 of SEQ ID NO. 4 in the Sequence List.

The coding gene for any of the above-described fusion proteins is also within the protecting scope of the present invention.

The above-mentioned coding is a coding sequence represented by sequence 3 in the Sequence List;
The recombinant vectors, recombinant bacteria or transgenic cell lines containing the above-described genes are also within the protecting scope of the present invention.

The above-described recombinant vector is a recombinant expresion vector obtained by inserting the above gene into the topoiso-site of pEASYE2 plasmid. The recombinant bacterium is recombinant *E. coli* or recombinant yeast.

The use of the fusion protein or gene mentioned above in the preparation of drugs for preventing and/or treating diseases caused by Shiga toxin or Shiga toxin-producing pathogenic bacteria are also within the protection scope of the present invention.

Further, the above-described Shiga toxin-producing pathogenic bacterium is EHEC 0157: H7.

### Brief Description of the Figure

Figure 1 is a PCR electrophoretogram showing the identification of the recombinant bacteria pSAmB/*E.coli* TransB (DE3), wherein lane 1 is the PCR amplified product of *sAmB* gene and lane M is the DL2000 DNA marker.
Figure 2 shows the SDS-PAGE and Western blot results about the expressed products and purified products of the recombinant SAmB expressed in prokaryotic system, wherein lanes 1 and 3 are the lysate of uninduced bacteria, lane 2 shows the
   supernatant of the lysed induced bacteria, lane 4 shows the precipitation of lysed bacteria, lane 5 shows the protein marker of low molecular weights, lane 6 shows the purified SAmB product, lanes 7 and 8 respectively show the Western blot results of purified SAmB products using anti-Stx1B polyclonal antibody (produced in chickens) or anti-Stx2A polyclonal antibody (produced in rabbits).

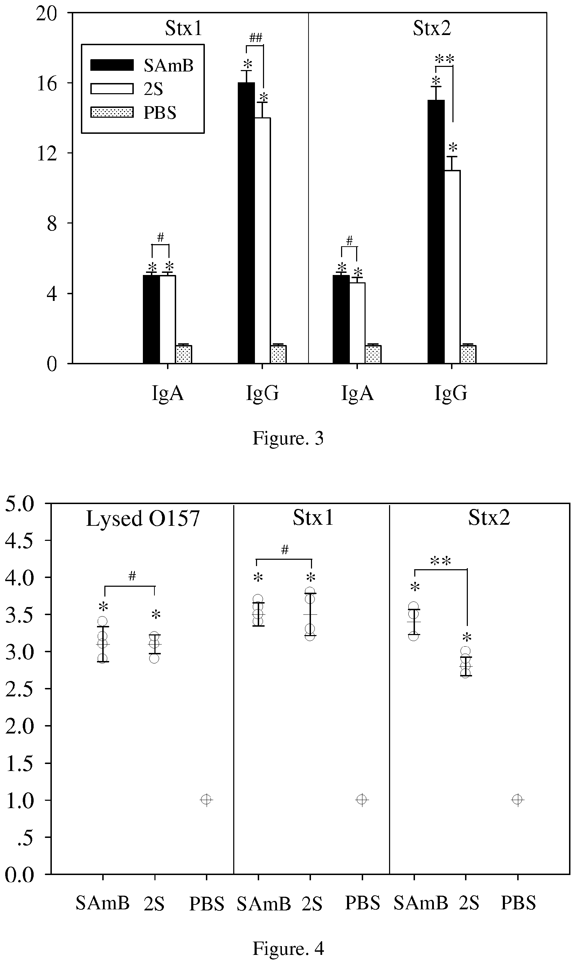

Figure 3 shows the antibody titer in the serum of immunized mice by 14 days after the last immunization, wherein antigens used for detection are Stx1 and Stx2 respectively. Mice were divided into three groups, wherein the black columns represent the group with SAmB as the immunogen, the blank columns represent the group with 2S as the immunogen and the grid columns represent the group with PBS as the immunogen. The vertical axis is the logarithm value of the ELISA titer of the serum antibody to base 2. The horizontal axis represents that antibodies in the serum are IgA or IgG, respectively. The mice in the placebo group has very low titer of serum IgA and IgG (<2) while the immunized groups have relative high titers. When compared to the placebo control PBS group, the symbol * denotes P<0.01 and # denotes P>0.05. When compared to the 2S group, the symbol **denotes P<0.01 and ## denotes P<0.05.
Figure 4 shows the test on neutralization titers of the immune serums against the supernatant of lysed 0157: H7 88321 bacteria, Stx1 and Stx2, wherein each value is obtained from a mixture of three serum samples. The vertical axis is the logarithm of neutralization titer to base 10. When compared to the placebo control PBS group, the symbol * denotes P<0.01. When compared to the 2S group, the symbol ** denotes P<0.01 and # denotes P>0.05. The experimental results revealed that immune serum of SAmB group could better neutralize Stx2 and had a higher neutralization titer (10^{3.4}) than the 2S group (10^{2.8}).

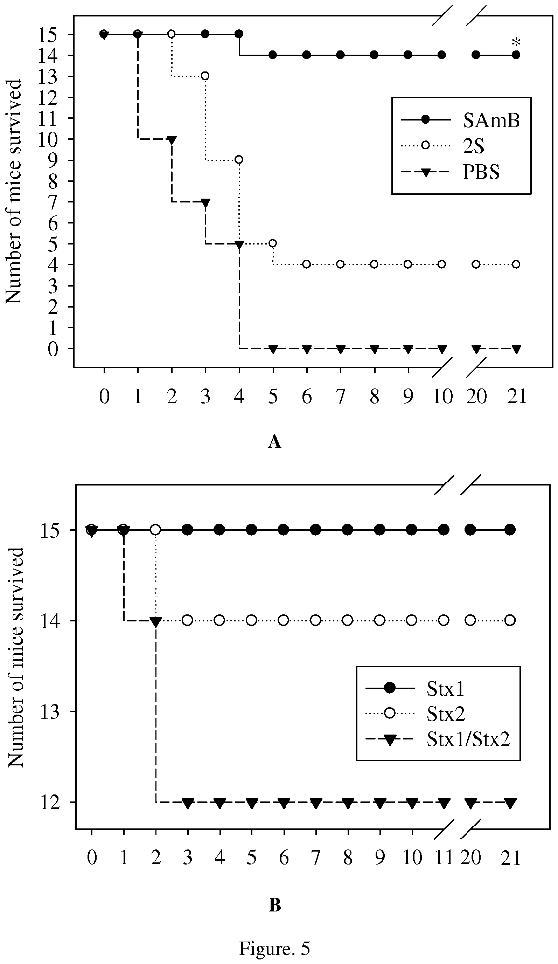

Figure 5 shows the protective effect against bacteria in immunized mice. Figure A shows the experimental results of immunized mice challenged with lysed *E.coli* 0157: H7 of a dose of 10 LD50. Figure B shows the results of immunized mice challenged with 10 LD50 of Stx1 or 10 LD50 of Stx2 or the mixture of 10 LD50 of Stx1 and 10 LD50 of Stx2. The vertical axis represents the number of mice in each group and the horizontal axis represents the number of days from the application of toxins (Day 0).

### Detailed Embodiments

The present invention will be further illustrated by referring to specific examples but the present invention is not limited to the following examples.

The methods used in the examples are conventional means if not indicated otherwise.

### Example 1. Construction, expression and purification of recombinant fusion protein SAmB

The bacterial strains and plasmids used in the present invention were shown in Table 1 and the primers used were shown in Table 2. The HisTRAP FF™ (a Ni affinity chromatographic column) and ÄKTA FPLC system for purification were both purchased from GE.

**Table 1. Bacterial strains and plasmids used in the experiments**

| Strains and plasmids | Characteristics | Sources |
|---|---|---|
| *E. coli* strain | | |
| O157:H7 88321 | Toxigenic strain, dual toxins-producing strain producing both Stx1 and Stx2 | Purchased from ATCC |
| O157:H7 EDL 933 | an international standard strain, dual toxins-producing strain producing both Stxland Stx2 | Purchased from ATCC |
| Trans5α | F⁻ ϕ80d *lacZ*ΔM15 Δ(*lac*ZYA-*arg*F) U169 *end*A1 *rec*A1 *hsd*R17(r_{K}⁻, m_{K}⁺) *sup*E44 *λ*-*thi-*1 *gyr*A96 *relA*1 *pho*A | Transgen |
| TransB (DE3) | *F⁻ omp*T *hsd*S_{B} (r_{B}⁻m_{B}⁻)*gal dcm lac*Y1 *ahp*C (DE3) *gor*522::*Tn*10 *trx*B (Kan^{R}, Tet^{R}) | Transgen |
| Vector | | |
| pEASYE2 | *E. coli* expression vector (Amp^{r}) | Transgen |

**Table 2. Pimers used in the experiments**

| Primers | Sequences (5'-3') ^{a, b} | Homologous regions |
|---|---|---|
| 1 | | Forward primer for Stx2A |
| 2 | | Reverse primer for Stx2A |
| 3 | | Forward primer for Stx1B |
| 4 | | Reverse primer for Stx1B |
| 5 | | The primer for mutation (77Y→77S) in Stx2A, used in combination with primer 6 |
| 6 | | The primer for mutation (77Y→77S) in Stx2A, used in combination with primer 5 |
| 7 | | The primer for mutation (167E→167Q and 170R→170L) in Stx2A, used in combination with primer 8 |
| 8 | | The primer for mutation (167E→167Q and 170R→170L) in Stx2A, used in combination with primer 7 |

| | | |
|---|---|---|
| ^{a}: Restriction endonuclease cleavage sites (underlined) ^{b}: Linker Sequence (bold font) | | |

### 1. Constrution of the recombinant expression vector

### 1) Obtaining of the coding gene for the fusion protein

The gene of the Shiga toxin type-II A subunit (*Stx2A*) (represented by SEQ ID NO. 1 in the Sequence List, the SEQ ID NO. 1 being capable of encoding the protein represented by SEQ ID NO. 5) was amplified from the genome of EHEC O157: H7 EDL933 (see the characteristics of the strain and its source in Table 1) using primers 1 and 2. The gene of the Shiga toxin type-I B subunit (*Stx1B*) (represented by SEQ ID NO. 2 in the Sequence List) was amplified using primers 3 and 4 (see the primers in Table 2).

Two fragments were amplified from *Stx2A* gene resepectively using two primer pairs, primers 1 and 6 as one pair and primers 2 and 5 as another. The obtained fragment mixture was used as the template (primers 1 and 2 were used as the primers) and then the gene for a *Stx2A* with one amino acid mutated was obtained by overlapping PCR.

Thereafter, two fragments were amplified from the gene with one base mutated using a two primer pairs, primers 1 and 8 as one pair and primers 2 and 7 being another. The obtained fragments was mixed and used as the template and primers 1 and 2 were used as the primer pair. An overlapping PCR was performed to obtain a *Stx2A* gene named *Stx2Am* with 3 amino acids mutated. At last, the *Stx2A* gene with 3 amino acids mutated (*Stx2Am*) was mixed with *StxlB* gene, which would be used as the template later. Then, a gene encoding fusion protein was obtained by overlapping PCR (using primers 1 and 4) and named *SAmB.* 2) Construction of the recombinant expression vector

The above-amplified *SAmB* after purified was ligated into the expression vector pEASYE2 (the vector was a topoiso-vector which did not need any restriction endonuclease cleavage site) (the detailed operations see the instruction from Transgen) to construct a recombinant expression vector called *pSAmB.*

Subsequently, *pSAmB* was transformed into *E.coli* DH5a and sequenced by Beijing Sunbiotech Co., Ltd. The sequencing results indicated that the obtained sequence was completely the same as previously designed.

Also, the sequencing results demonstrated that p*SAmB* fragment represented by SEQ ID NO. 3 in the Sequence List had been inserted in the the topoiso-site of pEASYE2.

In SEQ ID NO. 3, the nucleic acid sequence of *Stx2A* gene with three-sites mutations (i.e., Stx2Am) was represented by nucleotides 1-891 of SEQ ID NO. 3 in the Sequence List and that of *StxlB* gene was represented by nucleotides 907-1113 of SEQ ID NO. 3 in the Sequence List (i.e., SEQ ID NO. 2). The nucleic acid sequence represented by SEQ ID NO. 3 can encode the protein represented by SEQ ID NO. 4 in the Sequence List, the protein being named SAmB. 2. Construction of the engineered bacteria, expression induction and purification 1) Construction of the engineered bacteria

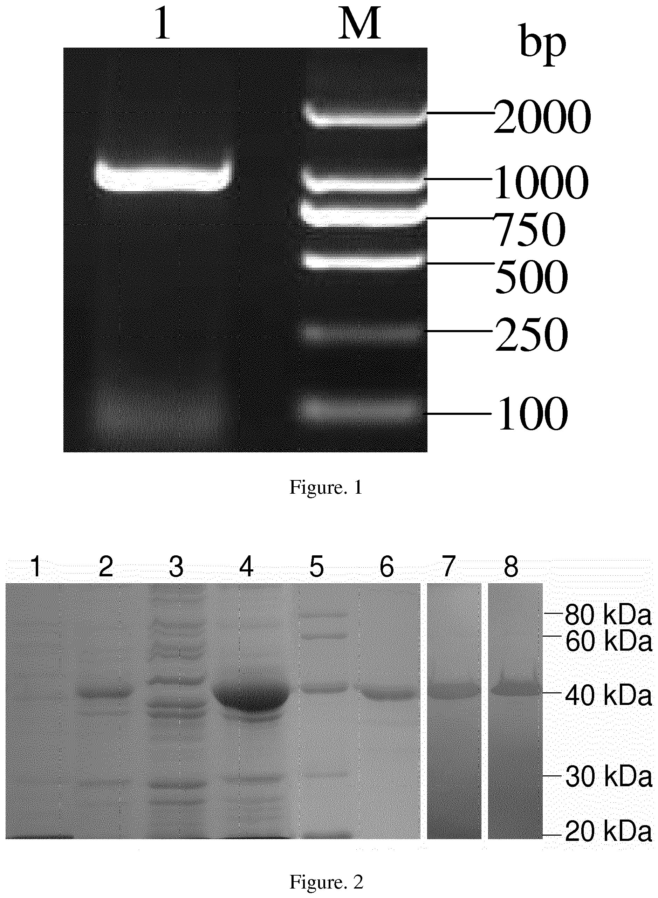

The above-confirmed plasmid p*SAmB* was transformed into *E.coli* TransB (DE3) to construct an engineered bacteria strain. PCR was used to identify the clones of the engineered bacteria strain by using primers 1 and 4 (Figure 1). A gene product of 1113bp was amplified from the positive clones which was consistent with sAmB gene in size indicating that *pSAmB* had been successfully transformed into *E.coli* TransB (DE3).

### 2) Induced expression and purification

The engineered bacteria strain was inoculated into liquid LB medium containing ampicillin (100 µg/mL) and shaked overnight at 37 °C. The next day, the bacteria were subcultured to liquid LB medium in a ratio of 1:100 to further grow with shaking at 37 °C until the culture reached OD600 of 0.4-0.6. Then, IPTG was added to a final concentration of 1 mM. Culture was continued for another 4 hours in water bath shaking bed with 150r/min at 37°C. Bacteria were centrifuged at 6000 r/min for 15 minutes at 4°C and resuspended in PBS (the concentration of EDTA was 1 mM, pH7.4). After lysis by ultrasonication, the bacterial lysates were centrifuged at 1000 g for 15 minutes. Then, the obtained precipitates were collected.

The isolated inclusion bodies were denatured in purification buffer A (8M urea, 20 mM sodium phosphate, 0.5 M NaCl, 40 mM imidazol, pH 7.4) wherein the purification step was performed according to the instruction of the Ni-NTA purification column (Amersham). Then, the purified products were dialysed with urea gradient (8M, 6M, 4M, 2M, 1M) and renatured. The products were finally dialysed into PBS.

Analysis using SDS-PAGE was done as follows. The recombinant bacteria after induction and the purified proteins were collected and identified by 15% SDS-PAGE electrophoresis. The results showed that a protein band of 40kDa was found in the recombinant bacterial sample, the molecular weight of which was the same as expected molecular weight of SAmB (Figure 2). The thin layer chromatography scan indicated that the expressed target proteins accounted for about 50% of the total bacterial proteins. It was also indicated that the purity of SAmB purified by Ni-NTA was 95% or above which can be used in immunizing animals to test its immunogenicity.

### 3) Antigenicity of SAmB

Western blot was done on the above purified SAmB using anti-Stxl and anti-Stx2 monoclonal antibodies (purchased from Biodesign). HRP-conjugated IgG (produced in chickens or rabbits) was used as the secondary antibody, and DAB was used in the colorimetric reaction. A specific band of 40 kDa was found in positive clones, the molecular weight was with the same as expected one (Figure 2, lanes 7 and 8), indicating that the recombinant SAmB had a good immunoreactivity.

### Example 2. Immunogenicity of fusion protein SAmB, and the anti-adhesion activities of immune serum in vitro

The reagents used in the present example were listed below. The MacConkey agar containing sorbitol, MTT and Giemsa stain were all purchased from Beijing Kehaijunzhou Company (China). The DMEM media, fetal bovine serum and dual antibodies were respectively purchased from GIBCO, Biochrom and Hyclone. The Cefixime granules were the product of the Pharmaceutical Factory, Guangzhou Baiyun Mountain. The potassium tellurite (1%) was purchased from Beijing Land Bridge Technology Co., Ltd. Animals were purchased from the Laboratory Animal Center of the Academy of Military Medical Sciences. The Freund's complete adjuvant and Freund's incomplete adjuvant were purchased from Sigma.

The present invention used 2S protein as the positive control to evaluate the immunogenicity and protective efficacy of SAmB. The 2S protein was a fusion protein obtained by concatenating Stx2B and Stx1B with GGGGS as a linker, that is,
*Stx2B-StxlB.* This protein was constructed by the inventors and the preparing process had been published in Vaccine (Gao X, Cai K, Shi J, Liu H, Hou XJ, Tu W, Xiao L, Wang Q, Wang H. Immunogenicity of a novel Stx2B-StxlB fusion protein in a mice model of Enterohemorrhagic Escherichia coli 0157: H7 infection. Vaccine, 2009, 27 (14): 2070-6). Specifically, the producing method was as below: Stx1B gene was amplified from EHEC 0157:H7 EDL933 by PCR using primer PI
(5' - AAGTGC AGTTT AAT AATGACGGAGGAGGAGGTTCTCCTGATTGTGT AACTG GAAA-3') and primer P2 (5'-GCGGCCGCACGAAAAATAACTTCGCTGAAT-3'). and stx2B was amplified using primer p3
(5' -CCCCCCCCATATGGCTAAAGGTAAAATTGAGTTTTCCAAG-3') and primer p4 (5'-GTCATTATTAAACTGCACTTCAGCAAATCCGGAGCCTGATTC-3'). The 2S protein was ligated by Splicing Overlap Extension PCR, i.e., primers p3 and p2 were used to amplify the full-length 2s gene. Then, the double-chained PCR product was purified and ligated into pMD18-T. The pMD18-T was transformed into *E. coli* DH5α which was then sequenced in Invitrogen (Beijing). The confirmed plasmid was digested by two restriction endonucleases and ligated with pET22b(+) vector (purchased from Novagen) treated by the same two endonucleases to construct a recombinant expression vector p2S. Then, the p2S was transformed into *E. coli* BL21 (DE3). The obtained engineering bacteria with p2S were expressed under IPTG induction. The expressed inclusion bodies were denatured, renatured and then purified by Q column to obtain the 2S protein.

The two toxins, Stx1 and Stx2, were used in the present invention as the antigens to test the immunogenicity of SAmB. The preparation methods were similar for these two proteins, the details of which had been published (Tu W, Cai K, Gao X, Xiao L, Chen RC, Shi J, Liu H, Hou XJ, Wang Q, Wang H, Improved production of holotoxin Stx2 with biological activities by using a single-promoter vector and an auto-induction expression system. Protein Expr. Purif, 2009, 67(2): 169-174). Wherein Stx2 was prepared as described below. Firstly, the full-length Shiga toxin stx2 gene was amplified from EHEC 0157:H7 using primers p5
(5'-GGGGGGAATTCATGAAGTGTATATTATTTAAATGGG-3') and p6 (5 ' - TTTTTTGTCGACTTAGTGGTGGTGGTGGTGGTGGTC ATTATTAAACTGC ACT TCAG-3'). Then, the Stx2 was sequenced using clone vector pEASY-Tl. The sequencing results suggested indicated that the nucleic acid of the amplified product was actually the same as the nucleotide from No. 1352290-1353527 of the nucleotide of Genbank NC_002655. A recombinant strain pET32a-stx2/plysS was constructed, which contained by two open reading frames strategy with a single promoter. Specifically, the amplified product was inserted into pET32a digested with restriction endonucleases *EcoR* I and *Sal* I to construct a recombinant plasmid pET32a-stx2. Then this plasmid was transformed into *E. coli* BL21 (DE3) plysS strain (purchased from Transgen) to obtain construct a recombinant strain pET32a-stx2/plysS. The recombinant plasmid pET32a-stx2 had one T7 promoter and the full-length clone of Shiga toxin stx2 contained two open reading frames (stx2a and stx2b). IPTG was added to a final concentration of 1 mmol/L. Then, the cells grew at 30 °C in water bath under shaking condition for 5 hours, leading to the highly effective expression of Stx2. Stx2 with 90% purity or higher more was obtained after treatment in Ni-HisTrap FF column and the obtained Stx2 showed biological activity. Details of related experiments have been published (Tu W, Cai K, Gao X, Xiao L, Chen RC, Shi J, Liu H, Hou XJ, Wang Q, Wang H, Improved production of holotoxin Stx2 with biological activities by using a single-promoter vector and an auto-induction expression system. Protein Expr. Purif. 2009, 67(2): 169-174).

The producing method of Stx1 was similar with the above producing method of Stx2 with the difference in using the primers of p7 (5-ggggggggaattcatgaaaataattatttttagagtgctaacttttttc-3) and p8 (5-gtcgactcagtggtggtggtggtggtgacgaaaaataacttcgctgaat-3). The nucleotide sequence of the amplified product was actually the same as from No.2995757-2996977 of the nucleotide of Genbank NC_002655. The *E. coli* TransB (DE3) was chosen as the host bacteria in the construction of pET32a-stxl/E.coli TransB(DE3). Stx1 with 90% purity or higher was obtained after treatment with Ni-HisTrap FF column and the obtained Stx1 showed a biological activity.

### 1. The titer of serum in mice immunized with SAmB protein detected by Enzyme-linked immunosorbent assay (ELISA)

### 1) Animal immunization

Female BALB/c mice (15- 17g) were randomly divided into five groups and according to the immunizing antigens, i.e., they were divided into (1) SAmB group, (2) 2S group and (3) PBS group. These antigens were intraperitoneally injected. The first immunization (referred to as Day 0) was taken with equal volumes of mixture of the antigens and Freund's complete adjuvant (total volume: 100 µl). The boosts immunization was taken with equal volumes of mixture of the antigens and Freund's incomplete adjuvant (at Day 14 and Day 28). Immunization was performed for three times with a two-week interval. The final concentration of the antigen for the first immunization was 25 µg and the final concentration of the antigent for the boosts immunization was 50µg.

### 2) Test of the titer of serum in immunized mice

Blood was collected from the eyepit 14 days after the last immunization (Day 42) and the serum was separated from the blood. The serum titer was detected by ELISA by using anti-Stxl and anti-Stx2 antibodies. Specifically, purified proteins Stx1 and Stx2 were respectively used to coat the plates (100 ng/well) and incubated at 4°C overnight. After washes by PBST, BSA was used for blocking. After washes, mouse serums with serial 2-fold dilution were added to the wells (the first one was diluted at a ratio of 1:50), wherein a double recovery was set for each well. After washes, HRP-conjugated anti-mouse IgA or IgG was used as the secondary antibody (Pierce, diluted with a ratio of 1:10⁵). Absorption was read after the colorimetric reaction was stopped. As shown in
Figure 3, the antibody titers against Stx1B and Stx2A in SAmB group were both higher than those in 2S group.

### 2. Test on the neutralizing titer of mice serum

Neutralization titers of the serum on Stx1 and Stx2 in immunized mice were measured. The 50% cytotoxic dose of Stx1 and Stx2 to Vero cells (ATCC) was tested in a preliminary experiment. The dose of the toxin in the detection of neutralization titers was 5CD50. Respectively, 500 pg of Stx1 and 20 ng of Stx2 were referred to as 1CD50. The supernatant of the lysed O157: H7 bacteria was obtained by centrifugating EHEC O157: H7 bacteria cultured overnight in Luria-Bertani medium (LB, Oxoid LTD, Basingstok, Hampshire, England) at 37°C, wherein 5×10⁴ CFU of supernatant of lysed O157: H7 bacteria can be referred to as 1CD50.

The extent of dilution was defined as the neutralization titer of the serum when 50% of the cells were protected from 5CD50 of toxins. The detailed testing procedure was shown as below: serums gradiently diluted with DMEM medium (with 2% of FBS) was respectively incubated with 5CD50 of the supernatant of lysed O157: H7 88321 bacteria, Stx 1 and Stx2 at 37°C for 1 hour. Then, the mixture was added into the 96-well plate and cultured in monolayered Vero cells (10⁶/well) in a CO₂ incubator at 37°C for 4 hours. After washed by PBS, fresh DMEM medium (with 0.5 mg/ml MTT) was added to continue the culture for another 4 hours. Then, impurities were washed away by PBS and 150 µl of DMSO was added to each well. Incubation was done for 10 min, and then the absorbance was read at 570 nm in a microplate reader. Each sample had three repeats and the test was independently repeated twice.

As shown in Figure 4, the results demonstrated that specific neutralizing antibodies against the supernatant of the lysed O157: H7 bacteria, Stx1 and Stx2 were generated in the serums after 14 days from the last immunization of SAmB, wherein neutralization titer against Stx2 was significantly higher in SAmB group than 2S group.

### Example 3. Immune protection effect of fusion protein SAmB against EHEC O157: H7 infection

The procedure for animal immunization was the same as described in Example 2. As two toxins, Stx1 and Stx2 proteins were used to attack SAmB immuned mice. The preparation method of these two proteins was the same as described in Example 2.
1. Protective effect in the mice of immunization group against lethal dose of lysed EHEC O157:H7 bacteria
   The lysed O157: H7 bacteria of 10LD50 (1LD50=10⁷CFU) were used to attack mice who had received three times of SAmB immunization. There were 15 mice in each group. The survival rate of mice was counted throughout the 21 days. The results demonstrated that 93.3% of mice were protected in SAmB group (14/15) while all mice died in placebo (PBS) group (0/15) (Figure 5A) and 26.7% survived in 2S immunization group (4/15).
2. Protective effect in the mice of immunization group against lethal dose of Stx
   Mice received three times of SAmB immunization. There were 15 mice in each group and the survival rate of mice was counted during the 21 days. The results demonstrated that 100% of mice were protected against 10LD50 (15µg) of Stx1 (15/15) and 93.3% of mice were protected against 10LD50 (60ng) of Stx2 (14/15). Meanwhile, 80% of mice were protected when the mixture of 10LD50 of Stx1 and 10LD50 of Stx2 were used in attacking (12/15) (Figure 5B).

### Industrial Applications

The experiments indicated that the fusion protein SAmB provided in the present invention had a good immunoreactivity and the antibody titers against Stx1B and Stx2A were both better in SAmB group than 2S group (i.e., the control group). Further, specific neutralizing antibodies against the supernatant of the lysed O157: H7 bacteria, Stx1 and Stx2 were generated in the serums after 14 days from the last immunization of SAmB, wherein neutralization titer against Stx2 was significantly higher in SAmB group than 2S group. In another aspect, the animal experiments implied that under the attack of high lethal dose of lysed EHEC O157:H79 bacteria, 93.3% of mice were under protection in SAmB group (14/15) while all mice died in placebo (PBS) group (0/15) and 26.7% survived in 2S group (4/15). In addition, in the SAmB group, 100% of mice were under protection against high lethal dose of Stx1 and 93.3% of mice were protected against high lethal dose of Stx2. Further, 80% of mice were under protection in the SAmB group when mixture of high lethal dose of Stx1 and high lethal dose of Stx2 were used.

## Claims

1. A fusion protein, comprising on the N-terminal a Shiga toxin type-II A subunit mutant protein and on the C-terminal a Shiga toxin type-I B subunit protein, the Shiga toxin type-II A subunit mutant protein is an inactivated protein obtained by mutating amino acid residues at active sites of Shiga toxin type-II A subunit, wherein the amino acid sequence is represented by SEQ ID NO. 4 in the Sequence List.

2. The fusion protein of claim 1, **characterized in that**:
the amino acid residues at active sites were amino acids No. 77, 167 and 170 in the Shiga toxin type-II A subunit, and the amino acid sequence of the A subunit of type-II Shiga toxin is represented by SEQ ID NO. 5 in the Sequence List.

3. The fusion protein of claims 1 or 2, **characterized in that**:
the amino acid sequence of the mutant protein of A subunit mutation of type-II Shiga toxin is represented by amino acids 1-297 of SEQ ID NO. 4 in the Sequence List, and the amino acid sequence of the Shiga toxin type-I B subunit protein is represented by amino acids 303-371 of Sequence 4 in the Sequence List.

4. A coding gene for encoding the fusion protein of any one of claims 1-3.

5. The coding gene of claim 4, **characterized in that**:
the coding gene is a coding sequence represented by SEQ ID NO. 3 in the Sequence List.

6. A recombinant vector, a recombinant bacterium or a transgenic cell line containing the genes of claim 4 or 5.

7. The recombinant vector or the recombinant bacterium of claim 6, **characterized in that**:
the recombinant vector is a recombinant expression vector obtained by inserting the gene of claim 4 or 5 into the topoiso-site in the pEASYE2 plasmid, and the recombant bacterium is a recombinant *E. coli* or a recombinant yeast.

8. The use of the fusion protein of any one of claims 1-3 or the gene of claim 4 or 5 in the drug preparation for preventing and/or treating diseases caused by Shiga toxin or Shiga toxin-producing pathogenic bacterium.

9. The use of claim 8, **characterized in that**:
the Shiga toxin-producing pathogenic bacterium is EHEC O157: H7.

## Patentansprüche

1. Ein Fusionsprotein, umfassend, am N-Terminus, ein Shiga-Toxin Typ-II A Untereinheitsmutantenprotein und, am C-Terminus, ein Shiga-Toxin Typ-I B Untereinheitsprotein, wobei das Shiga-Toxin Typ-II A Untereinheitsmutantenprotein ein inaktiviertes Protein ist, erhalten durch Mutation von Aminosäureresten an aktiven Zentren der Shiga-Toxin Typ-II A Untereinheit, wobei die Aminosäuresequenz dargestellt ist durch SEQ ID NO. 4 in der Sequenzliste.

2. Das Fusionsprotein gemäß Anspruch 1, **gekennzeichnet dadurch, dass**:
die Aminosäurereste an den aktiven Zentren die Aminosäuren Nr. 77, 167 und 170 in der Shiga-Toxin Typ-II A Untereinheit waren und die Aminosäuresequenz der A Untereinheit des Typ-II Shiga-Toxins dargestellt ist durch SEQ ID NO. 5 in der Sequenzliste.

3. Das Fusionsprotein gemäß Ansprüchen 1 oder 2, **gekennzeichnet dadurch, dass**:
die Aminosäuresequenz des Mutantenproteins der A Untereinheitsmutation des Typ-II Shiga-Toxins dargestellt ist durch Aminosäuren 1-297 von SEQ ID NO. 4 in der Sequenzliste und die Aminosäuresequenz des Shiga-Toxin Typ-I B Untereinheitsproteins dargestellt ist durch Aminosäuren 303-371 der Sequenz 4 in der Sequenzliste.

4. Ein kodierendes Gen zum Kodieren des Fusionsproteins gemäß einem der Ansprüche 1-3.

5. Das kodierende Gen gemäß Anspruch 4, **gekennzeichnet dadurch, dass**:
das kodierende Gen eine kodierende Sequenz dargestellt durch SEQ ID NO. 3 in der Sequenzliste ist.

6. Ein rekombinanter Vektor, ein rekombinantes Bakterium oder eine transgene Zelllinie enthaltend die Gene gemäß Anspruch 4 oder 5.

7. Der rekombinante Vektor oder das rekombinante Bakterium gemäß Anspruch 6, **gekennzeichnet dadurch, dass**:
der rekombinante Vektor ein rekombinanter Expressionsvektor ist, erhalten durch Insertion des Gens gemäß Anspruch 4 oder 5 in die Topoiso-Region in dem pEASYE2 Plasmid, und das rekombinante Bakterium ein rekombinantes *E. coli* oder eine rekombinante Hefe ist.

8. Die Verwendung des Fusionsproteins gemäß einem der Ansprüche 1-3 oder des Gens gemäß Anspruch 4 oder 5 in der Arzneimittelherstellung zur Prävention und/oder Behandlung von Erkrankungen, die durch das Shiga-Toxin oder ein Shiga-Toxin-produzierendes, pathogenes Bakterium hervorgerufen werden.

9. Die Verwendung gemäß Anspruch 8, **gekennzeichnet dadurch, dass**:
das Shiga-Toxin-produzierende, pathogene Bakterium EHEC O157:H7 ist.

## Revendications

1. Protéine de fusion, comprenant sur la terminaison N une protéine mutante de sous-unité A de toxine de Shiga type II et sur la terminaison C une protéine de sous-unité B de toxine de Shiga type I, la protéine mutante de sous-unité A de toxine de Shiga type II est une protéine inactivée obtenue en mutant des résidus d'acides aminés au niveau de sites actifs de la sous-unité A de toxine de Shiga type II, dans laquelle la séquence d'acides aminés est représentée par SEQ ID N°4 dans la liste de séquences.

2. Protéine de fusion selon la revendication 1, **caractérisée en ce que** :
les résidus d'acides aminés au niveau de sites actifs étaient les acides aminés N°77, 167 et 170 dans la sous-unité A de toxine de Shiga type II, et la séquence d'acides aminés de la sous-unité A de la toxine de Shiga type II est représentée par SEQ ID N°5 dans la liste de séquences.

3. Protéine de fusion selon les revendications 1 ou 2, **caractérisée en ce que** :
la séquence d'acides aminés de la protéine mutante de la mutation de sous-unité A de toxine de Shiga type II est représentée par les acides aminés 1 à 297 de SEQ ID N°4 dans la liste de séquences, et la séquence d'acides aminés de la protéine de toxine de sous-unité B de Shiga type I est représentée par les acides aminés 303 à 371 de la séquence 4 dans la liste de séquences.

4. Gène codant destiné à coder la protéine de fusion de l'une quelconque des revendications 1 à 3.

5. Gène codant selon la revendication 4, **caractérisé en ce que** :
le gène codant est une séquence codante représentée par SEQ ID N°3 dans la liste de séquences.

6. Vecteur recombinant, bactérie recombinante ou lignée cellulaire transgénique contenant les gènes de la revendication 4 ou 5.

7. Vecteur recombinant ou bactérie recombinante selon la revendication 6, caractérisé(e) en ce que :
le vecteur recombinant est un vecteur d'expression recombinant obtenu par insertion du gène de la revendication 4 ou 5 dans le topoiso-site dans le plasmide pEASYE2, et la bactérie recombinante est une *E. coli* recombinante ou une levure recombinante.

8. Utilisation de la protéine de fusion de l'une quelconque des revendications 1 à 3 ou du gène de la revendication 4 ou 5, dans la préparation de médicament destiné à empêcher et/ou traiter des maladies provoquées par la toxine de Shiga ou une bactérie pathogène produisant la toxine de Shiga.

9. Utilisation selon la revendication 8, **caractérisée en ce que** :
la bactérie pathogène produisant la toxine de Shiga est EHEC 0157 :H7.
